# EUROPEAN PATENT SPECIFICATION

(11) **EP 2 920 780 B1**
(45) Date of publication and mention of the grant of the patent: **05.04.2023**
(21) Application number: 13794960.8
(22) Date of filing: 15.11.2013
(51) Int. Cl.: G09F 3/00, A24F 25/02, G09F 3/10, B65D 81/24, B65D 85/10

(54) **MOISTURE CONTROL LABEL**
FEUCHTIGKEITSREGELUNGSETIKETT
ÉTIQUETTE RÉGULANT L'HUMIDITÉ

(30) Priority: 16.11.2012 US 201261727482 P; 07.01.2013 GB 201300225
(43) Date of publication of application: 23.09.2015
(73) Proprietor: MM Newport Ltd., Bradford BD10 9TE (GB)
(72) Inventor: WARD, Bennett Clayton, Midlothian, Virginia 23113 (US); XIANG, Jian, Midlothian, Virginia 23113 (US); GATER, Jenny, Ripley Derbyshire DE5 3UP (GB); ALLEN, Philip John, Nottingham Nottinghamshire NG16 5JD (GB)
(74) Representative: Bates, Philip Ian
(86) International application number: PCT/GB2013/053015
(87) International publication number: WO 2014/076488

(56) References cited:
- EP-A1- 1 818 091
- WO-A1-94/25263
- WO-A1-94/25263
- WO-A1-03/031531
- WO-A1-03/031531
- US-A- 5 037 459
- US-A- 5 037 459
- US-B1- 6 871 419
- US-B2- 6 601 875

## Description

The invention relates to a self-adhesive moisture control label for product packaging which is capable of controlling the humidity of the atmosphere within the packaging. In particular, the invention relates to a self-adhesive moisture control label which can be used to humidify the atmosphere within product packaging, such as tobacco packaging, so as to prevent the product, such as tobacco, from drying out.

It may be desirable to control the moisture content (or humidity) of the atmosphere in which perishable products are stored in order to prevent them from deteriorating. Perishable products, such as tobacco and food, are often stored in a sealed container in an attempt to keep them fresh. However, the seal on such containers is never perfect, particularly if the containers are disposable. Therefore, over time, the perishable product may either dry out, or absorb water and become damp.

For example, in order to prevent tobacco from drying out it is desirable to store it in an atmosphere that is not too dry.

It is known to place a wet sponge in a cigar case in order to prevent the cigars from drying out. However, this is not appropriate for most other forms of tobacco, such as loose tobacco, as if the wet sponge comes into contact with the tobacco it may rot the tobacco.

It is therefore desirable to provide a means for controlling the moisture content of the atmosphere within product packaging.

WO9425263 relates to an oxygen-absorbing label and a moisture-sensitive label.

WO03031531 relates to adhesive articles which are capable of detecting sustained exposure to water.

According to an aspect of the invention there is provided a self-adhesive moisture control label for product packaging, comprising:
a water absorbent layer capable of absorbing water; and
an adhesive layer provided such that the label can be adhered to the packaging;
wherein the moisture control label is capable of controlling the humidity of the atmosphere within the packaging;
wherein water absorbed by the water absorbent layer can evaporate from the water absorbent layer so as to control the humidity of the atmosphere within the packaging; characterised in that the self-adhesive moisture control label comprises a base layer to which the water absorbent
layer is adhered on a front side and wherein the adhesive layer is provided on the rear side of the base layer; and a graphical indicium disposed on the base layer;
wherein in a dry state the water absorbent layer has a first opacity and wherein in a water saturated state the water absorbent layer has a second opacity which is lower than the first opacity, such that in use the graphical indicium becomes less visible as the water absorbent layer becomes drier, and becomes more visible as the water absorbent layer becomes wetter.

The water absorbent layer may be wetted by a user such that in use water evaporates from the surface so as to provide moisture (i.e. humidify) the atmosphere within the packaging. The label may therefore be used to prevent a product from drying out.

The water absorbed by the water absorbent layer is able to evaporate from the water absorbent layer so as to control the humidity of the atmosphere within the packaging.

The water absorbent layer may comprise a fibrous pad or layer. The fibrous pad or layer may be woven or non-woven (such as a mat). The fibrous pad may comprise polymer fibres. The fibrous pad may comprise polyolefin fibres and/or polyethylene terephthalate (PET) fibres and/or polyethylene (PE) fibres and/or nylon fibres and/or polyvinyl chloride (PVC) fibres and/or polylactic acid (PLA) fibres. In other embodiments the water absorbent layer may comprise cellulose and/or paper and/or cotton. The water absorbent layer may be porous. The water absorbent layer may comprise a hydrophilic material. The hydrophilic material may comprise a hydrogel. The water absorbent layer may comprise a sponge material. The water absorbent layer may be continuous.

The water absorbent layer may only be capable of absorbing less than 10ml, less than 8ml, less than 6ml, less than 4ml, less than 3ml, less than 2ml or less than 1ml of water. Further, the water absorbent layer may be capable of absorbing greater than 0ml, greater than 0.1ml, greater than 0.2ml, greater than 0.3ml, greater than 0.4ml, or greater than 0.5ml of water. The water absorbent layer may be capable of absorbing at least 0.6ml of water. The water absorbent layer may be capable of absorbing between 0.2-2ml, 0.3-1.5ml, 0.4-1ml or 0.5-0.8ml of water.

The water absorbent layer may have a thickness of less than 5mm, less than 4mm, less than 3mm or less than 2mm. The water absorbent layer may have a thickness of at least 0.1mm, at least 0.2mm, at least 0.4mm, at least 0.6mm, at least 0.8mm or at least 1mm. The water absorbent layer may have a thickness of between 0.1mm-5mm, 0.2mm-4mm, 0.3mm-3mm, 0.5mm-2mm, or 0.7mm-1.8mm.

The water absorbent layer may have an area (in plan view) of less than 100cm², less than 80cm², less than 60cm², less than 40cm², less than 20cm², less than 10cm², or less than 8cm². The water absorbent layer may have an area (in plan view) of greater than 0.1cm², greater than 0.2cm², greater than 0.5cm², greater than 1cm², greater than 1.5cm², greater than 2cm², greater than 3cm², greater than 4cm², greater than 5cm², greater than 6cm², or greater than 7cm². The water absorbent layer may have an area (in plan view) of between 0.2-20cm², 0.5-15cm², 1.5-10cm², or 3-8cm².

The water absorbent layer may have a density of less than 1g/cm³, less than 0.9g/cm³, less than 0.8g/cm³, less than 0.7g/cm³, less than 0.6g/cm³, less than 0.5g/cm³, less than 0.4g/cm³, less than 0.3g/cm³, or less than 0.2g/cm³. The water absorbent layer may have a density of greater than 0.01g/cm³, greater than 0.02g/cm³, greater than 0.03g/cm³, greater than 0.04g/cm³, greater than 0.05g/cm³, greater than 0.06g/cm³, greater than 0.07g/cm³, greater than 0.08g/cm³, greater than 0.09g/cm³, or greater than 0.1g/cm³. The water absorbent layer may have a density of between 0.01-1g/cm³, 0.02-0.9g/cm³, 0.03-0.8g/cm³, 0.05-0.7g/cm³, 0.08-0.5g/cm³, 0.09-0.3g/cm³, or 0.1-0.2g/cm³.

The base layer may be a uniform layer of material or may be a laminate structure. The adhesive layer, which may be continuous or discontinuous, may be provided on the rear side of the base layer. The base layer may be larger than the water absorbent layer such that a display region extends beyond the periphery of the water absorbent layer. Use instructions and/or commercial graphics may be provided on the display region of the base layer. The water absorbent layer may be located centrally with respect to the base layer. The base layer and/or the water absorbent layer may be circular, square, triangular or any suitable shape. If circular, the base layer and water absorbent layer may be concentric with one another.

The base layer may have a thickness of at least 10µm, at least 25µm, at least 50µm, at least 75µm or at least 100µm. The base layer may have a thickness of less than 1mm, less than 0.5mm, less than 0.25mm, or less than 0.15mm. The base layer may have a thickness of between 10µm-1000µm, 20µm-700µm, 50µm-500µm, 60-250µm, or 80µm-150µm.

The base layer may comprise polypropylene. The base layer may comprise mono-oriented polypropylene. The base layer may comprise polyethylene terephthalate (PET), polyethylene (PE) or polyvinyl chloride (PVC).

The water absorbent layer may be adhered to the base layer with a pressure sensitive adhesive, for example. The adhesive layer may comprise a pressure sensitive adhesive.

The opacity of the water absorbent layer varies as a function of the amount of water retained by the water absorbent layer. In a dry state the water absorbent layer has a first (high) opacity and in a saturated state the water absorbent layer has a second (low) opacity which is lower than the first opacity. In use, a graphical indicium disposed below the water absorbent layer becomes less visible as the water absorbent layer becomes drier, and becomes more visible as the water absorbent layer becomes wetter. This may provide a useful indication to the user as to the moisture content of the water absorbent layer. The graphical indicium is provided on the base layer. The graphical indicium may be any suitable graphic such as a logo, text, colour, marking, promotional information, symbol, or sign.

The invention also relates to a plurality of labels releasably adhered to a backing sheet, as disclosed in claim 7.

According to another aspect there is provided a wound reel of labels comprising a wound backing sheet with a plurality of labels releasably adhered to the backing sheet, as disclosed in claim 8.

According to yet another aspect of the invention there is provided product packaging defining a volume for containing a product according to claim 9, wherein a self-adhesive moisture control label is adhered to the packaging such that the label is disposed within the volume so that it can control the humidity of the volume.

The product packaging may be packaging for a tobacco product. The tobacco product may be loose tobacco or cigars or cigarettes or snuff or snus. The product packaging may comprise a tub having a container base defining a volume and a detachably attachable lid, wherein the label is adhered to the inside of the lid.

A method of manufacturing a wound reel of self-adhesive moisture control labels for product packaging comprises: applying an adhesive to the rear side of a continuous web of water absorbent material; releasably adhering the web of water absorbent material to a backing sheet; cutting a series of discrete water absorbent layers from the web of water absorbent material; removing the skeleton (or mask) of the water absorbent material; and winding the backing sheet into a reel.

The method may further comprise: applying an adhesive to the rear side of a continuous web of base material; releasably adhering the web of base material to a backing sheet; cutting a series of discrete base layers from the web of base material; removing the skeleton of the base material; and repeatedly removing a discrete water absorbent layer from its backing sheet and adhering to a discrete base layer to form a series of laminate self-adhesive moisture control labels.

It should be appreciated that the method steps may be performed in any suitable order and are not limited to the order presented herein.

Embodiments of the invention will now be described, by way of example, with reference to the accompanying drawings, in which:
Figure 1 schematically shows a plan view of a self-adhesive moisture control label;
Figure 2 schematically shows a cross-sectional view through a self-adhesive moisture control label;
Figure 3 schematically shows product packaging having a moisture control label adhered thereto;
Figure 4 schematically shows a moisture control label in a wet state; and
Figure 5 schematically shows a moisture control label in a dry state.

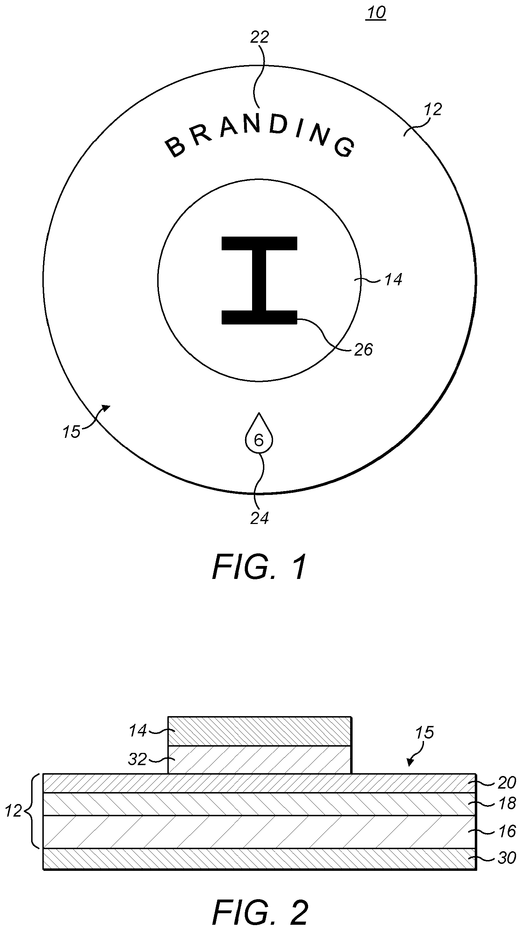

**Figures 1 and 2** show a self-adhesive moisture control label 10 for product packaging. In this particular embodiment the label 10 is intended to be adhered to product packaging so as to provide moisture to the atmosphere within the packaging. As will be described in detail below, the label 10 comprises a base layer 12 and a water absorbent layer 14 which is adhered to the upper side of the base layer 12. The lower side of the base layer 12 is provided with an adhesive layer 30 such that the label 10 can be adhered to product packaging. In use, a small amount of water is provided to the water absorbent layer 14 which is absorbed and retained by the water absorbent layer 14. The water retained by the water absorbent layer 14 is then able to evaporate from the water absorbent layer 14 so as to provide moisture to the atmosphere within the product packaging. The label 10 therefore acts to humidify the surrounding atmosphere.

The label 10 is substantially circular and comprises a circular base layer 12 of a first diameter, and a circular water absorbent layer 14 of a second diameter that is less than the first diameter. In this embodiment the diameter of the base layer 12 (and therefore the overall label 10) is approximately 50mm and the diameter of the water absorbent layer 14 is approximately 25mm. The base layer 12 and water absorbent layer 14 are concentric with one another and the base layer 12 extends beyond the periphery of the water absorbent layer 14 to form a display region 15. It should be appreciated that other shapes and dimensions could be used, and the base layer 12 and water absorbent layer 14 could be positioned differently with respect to one another. Although it has been described that the base layer 12 is larger than the water absorbent layer 14, in other embodiments the layers could be identically sized, or the base layer 12 could be smaller than the water absorbent layer 14.

As shown in Figure 2, the base layer 12 is a laminate structure comprising a lower film 16, a print layer 18 and an upper film 20. The print layer 18 is therefore sandwiched between the lower film 16 and the upper film 20 which acts as a protective layer. In this embodiment, the lower film 16 is a layer of white polypropylene having a thickness of approximately 60µm and the upper film 20 is a layer of clear polypropylene having a thickness of approximately 20µm. It should be appreciated that other materials or thicknesses could be used. It should be noted that in other embodiments the base layer 12 could be a single uniform layer of film. The print layer 18 is directly printed onto the lower film 16 and may comprise any suitable colours, graphics, designs or text. In this embodiment the print layer comprises a solid colour which extends over the entire surface of the lower film 16, branding information 22, instructions for use 24 and a graphical indicium 26. The branding information 22 and the use instructions 24 are printed in the display region 15 beyond the water absorbent layer 14, whilst the graphical indicium 26 is printed in a region underneath the water absorbent layer 14. The function of the graphical indicium 26 will be described in detail below. The lower surface of the lower film 16 (and hence the lower surface of the base layer 12) is provided with a layer of pressure sensitive adhesive 30. The adhesive layer 30 may either be a continuous or a discontinuous coating. The adhesive layer 30 allows the label 10 to be adhered to product packaging. In other embodiments the print layer 18 may be separate from the base layer 12.

The water absorbent layer is in the form of a fibrous pad 14 which can absorb and retain water, and from which water can evaporate. In this embodiment the fibrous pad 14 is non-woven and is made from matted polyolefin fibres. The diameter, thickness and density of the fibrous pad 14 may be chosen such that the fibrous pad can absorb a required volume of water. For example, a polyolefin fibrous pad 14 having a 25mm diameter, a thickness of 1.5mm and a density of between 0.1-0.2g/cm³ may absorb approximately 0.6ml of water. In another example, a polyolefin fibrous pad 14 having a 31mm diameter, a thickness of 1mm and a density of between 0.1-0.2g/cm³ may absorb approximately 0.6ml of water. The lower surface of the fibrous pad 14 is provided with a layer of pressure sensitive adhesive 32 which adheres the fibrous pad 14 to the upper surface of the upper film 20 of the base layer 12.

The polyolefin fibrous pad 14 permits visible light to pass through it. However, the opacity of the fibrous pad 14 is the function of the amount of water retained by it. In particular, when the fibrous pad 14 is completely dry it has a higher opacity, and when it is completely saturated with water it has a lower opacity. The Applicant believes that this phenomena is due to the fibres reflecting light when dry, but allowing light to pass through them when wet. As will be described in detail below, this means that in use, the graphical indicium 26 printed on the base layer 12 becomes less visible as the fibrous pad 14 dries out. This may provide a useful indicator to the use that the fibrous pad 14 needs to be wetted.

A particular benefit of the fibrous pad 14 is that it maintains its integrity and tends not to fragment. This may be particularly important as it may be important to prevent fragments of the fibrous pad 14 being mixed with the product which is contained within the product packaging.

Although it has been described that the water absorbent layer 14 is a polyolefin fibrous pad 14, it should be appreciated that other materials could be used. The water absorbent layer 14 could be any woven or non-woven fibrous material and could be made from materials other than polyolefin fibres such as polyethylene terephthalate (PET) fibres, polyethylene (PE) fibres, nylon fibres, polyvinyl chloride (PVC) fibres, or polylactic acid (PLA) fibres, for example. In some embodiment the water absorbent layer may comprise a mixture of polymer fibres, such as any combination of the polymer fibres listed above. Further, the water absorbent layer 14 could be a sponge-like porous material capable of absorbing water, or could be a hydrophilic material such as a layer of a hydrophilic gel. Essentially, the water absorbent layer 14 could be made from any suitable material that is capable of absorbing and retaining water, and which allows water to subsequently evaporate from it.

Self-adhesive moisture control labels 10 may be provided on a wound backing sheet as a reel of labels. The labels 10 may then be applied either manually, or automatically, or by a combination of the two to product packaging.

A reel of moisture control labels 10 may be manufactured by the following method:
A web of lower film material 16 is printed with the desired print layer 18 which continually repeats. A web of upper film material 20 is then applied over the print layer 18 to provide a protective barrier. A pressure sensitive adhesive layer 30 is then applied to the underside of the lower film material 16 and the laminate structure is releasably adhered to a web of backing material. The individual base layers 12 are then die cut from the laminate and the skeleton (or mask) is removed.

A web of water absorbent material 14, such as a web of polyolefin fibrous material, is coated on its lower surface with a pressure sensitive adhesive layer 32. This is web is then releasably adhered to a web of backing material. The individual water absorbent layers 14 (or fibrous pads) are then die cut from the web and the skeleton (or mask) is removed.

The individual base layers 12 and water absorbent layers 14 are then assembled together to form a moisture control label 10. Specifically, by machine, the individual water absorbent layers 14 are peeled from the backing sheet and applied to the upper surface of individual base layers 12 which remain on the backing sheet. The backing sheet, having the assembled base layers 12 and water absorbent layers 14, is then wound-up to provide a reel of self-adhesive moisture control labels.

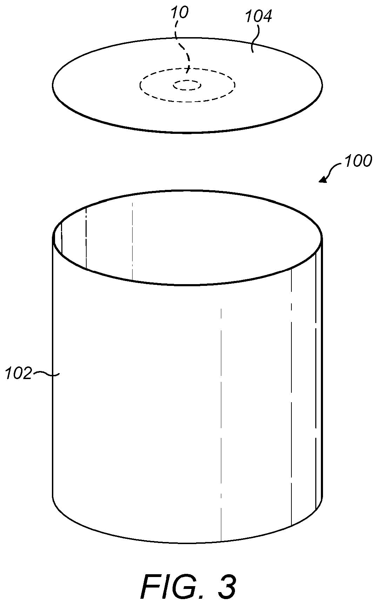

**Figure 3** shows product packaging in the form of a tub 100 for containing loose (or rolling) tobacco. The tub 100 is generally cylindrical and comprises a container base 102 having a cylindrical wall and a circular base, and a circular lid 104 which can be removably attached to the container base 102. The loose tobacco is stored within the container base 102 and the lid 104 is attached to the container base 102 in order to close the tub 100. Before the tub 100 has been opened for the first time a foil seal (not shown) hermetically seals the container base 102 and maintains the freshness of the tobacco. However, once this has been removed to obtain access to the tobacco for the first time, the lid 104 does not seal the container base 102 to the same extent.

It is desirable to prevent the tobacco from drying out as this can adversely affect its quality. Therefore, a self-adhesive moisture control label 10 is applied to the underside of the lid 104 so that it is disposed within the tub 100 when the lid 104 is attached to the container base 102. The label 10 may be adhered to the lid from a reel of labels 10 during manufacture of the packaging and may be applied either by hand, or by machine, or by a combination of the two. In another arrangement the label 10 may be disposed within the tub 100 with the tobacco and the consumer may have to adhere the label 10 to the inside of the lid.

Once tub 100 has been opened by removing the lid 104 and the foil seal (not shown), the consumer must wet the fibrous pad 14 of the label 10. The instructions for use of the label 10, together with branding information, is provided on the display region 15 of the base layer 12 which means that it is not necessary to provide any additional inserts or instructions for use. The use instructions 24 may direct the consumer to add a certain amount of water to the fibrous pad 14. In this embodiment, the consumer is directed to add 6 drops of water to the fibrous pad 14. The lid 104 is then attached to the container base 102 with the label 10 disposed within the tub 100. Over time, water evaporates from the fibrous pad 14 which acts to provide moisture to the atmosphere within the tub 100, humidifying the atmosphere, and preventing the tobacco from drying out. The label 10 therefore acts to maintain the quality of the tobacco.

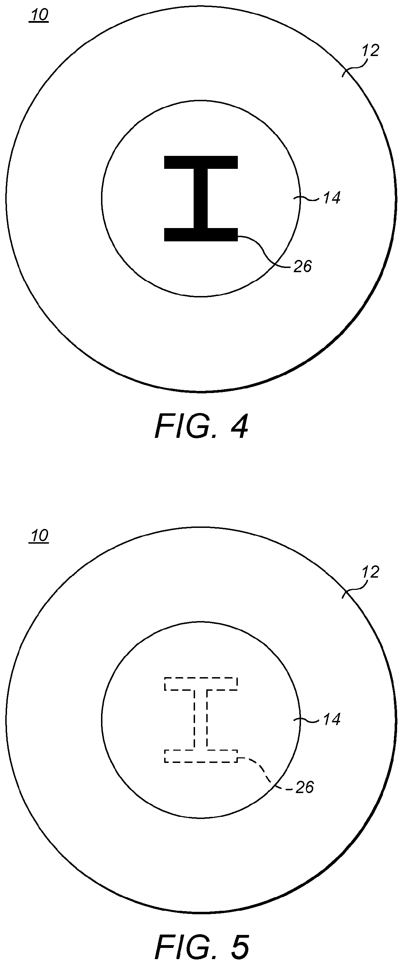

Referring now to **Figures 4 and 5**, as described above, the opacity of the water absorbent layer 14 (fibrous pad) changes as the volume of water retained by the fibrous pad 14 changes. In particular, the opacity of the fibrous pad 14 increases as the volume of water retained reduces. Therefore, when the fibrous pad 14 is saturated with water (i.e. when the consumer has added the prescribed number of drops of water), the opacity of the fibrous pad 14 is relatively low and therefore the graphical indicium 26 printed on the base layer 12 below the fibrous pad 14 is visible through the fibrous pad 14 (Figure 4). However, as the water evaporates from the fibrous pad 14 and the fibrous pad 14 becomes drier, the opacity of the fibrous pad increases. Therefore, as the fibrous pad 14 becomes drier the graphical indicium 26 becomes less visible, until the fibrous pad 14 completely dries out and the graphical indicium 26 becomes its least visible (Figure 5). It should be appreciated that even when dry, the graphical indicium 26 may be partly visible, if feint.

The change in opacity of the fibrous pad 14 in conjunction with the graphical indicium 26 disposed below it provides a useful indication to the consumer as to when it is necessary to add more water to the fibrous pad 14 in order to ensure that the atmosphere within the packaging is kept moist. The graphical indicium 26 may be any graphic and may include branding relating to the product. The change in opacity of the water absorbent layer 14 in conjunction with the graphical indicium 26 located below it provides a useful indicator.

In the above embodiment it has been described that the label 10 is applied to a tub 100 for loose tobacco. However, it should be appreciated that the label 10 could be used with other types of packaging for loose tobacco such as pouches. Further, the label 10 may be used with packaging for other tobacco products such as cigarettes, cigars, snuff or snus. As opposed to being applied to packaging during manufacture, the labels 10 may be sold separately to the end-user who may apply them to various types of packaging or container as appropriate. For example, a label 10 could be applied by an end-user to a tin within which is stored loose tobacco, or a case for storing cigars or cigarettes.

It has been described that the label 10 is used to prevent tobacco from drying out. However, it could be used to prevent other products such as perishable goods, processed foods, fresh foods, plants/flowers, agricultural compounds, seeds, pharmaceuticals, cosmetics, domestic or household chemicals, industrial chemicals, electronic components, instrumentation and devices, clothing or garments, footwear, luggage or bags, stationary or documentation, artwork or photography, jewellery, or musical instruments from drying out, and spoiling.

In another embodiment, the label may simply comprise a self-adhesive water absorbent layer 14, such as a fibrous pad, which can be adhered to product packaging. In such an embodiment the label would comprise the water absorbent layer 14 and the adhesive 32. The label could be applied to product packaging over a graphical indicium printed or otherwise provided on the packaging itself. As for the first embodiment, the water absorbent layer 14 would change in opacity as a function of the water retained by it and therefore the graphical indicium on the packaging would become more visible as the label gets wetter, and less visible as the label gets drier.

Due to the material properties of the fibrous pad, in particular the density and substantially uniform structure, the fibrous pad can be saturated with a specific known and reproducible volume of water. This allows a fibrous pad to be designed so as to hold a desired volume of water which is appropriate to the particular application. This ensures that the fibrous pad cannot be overfilled which helps to reduce the risk of damaging the product with which it is to be used.

In some embodiments, the material properties allow a relatively large fibrous pad to be manufactured, which holds a relatively small volume of water.

Where the term "layer" has been used in the foregoing specification, this should be understood as encompassing either a continuous or discontinuous layer (such as a grid or a series of discrete spots). For example, the adhesive layer 30 may be a continuous film of adhesive, or could be a series of stripes, or a grid, or spots.

### REFERENCE NUMERALS:

10 - label
12 - base layer
14 - water absorbent layer
15 - display region
16 -lower film
18 - print layer
20 - upper film
22 - branding
24 - use instructions
26 - graphical indicium
30 - adhesive
32 - adhesive
100 - tub
102 - container base
104 - lid

## Claims

1. A self-adhesive moisture control label (10) for product packaging, comprising:
a water absorbent layer (14) capable of absorbing water; and
an adhesive layer (30) provided such that the label (10) can be adhered to the packaging ;
wherein the moisture control label (10) is capable of controlling the humidity of the atmosphere within the packaging;
wherein water absorbed by the water absorbent layer (14) can evaporate from the water absorbent layer (14) so as to control the humidity of the atmosphere within the packaging; wherein the self-adhesive moisture control label comprises a base layer (12) to which the water absorbent layer (14) is adhered on a front side and wherein the adhesive layer (30) is provided on the rear side of the base layer (12);
**characterized in that**
a graphical indicium (26) is disposed on the base layer; **in that** in a dry state the water absorbent layer (14) has a first opacity and **in that** in a water saturated state the water absorbent layer (14) has a second opacity which is lower than the first opacity, such that in use the graphical indicium (14) becomes less visible as the water absorbent layer (14) becomes drier, and becomes more visible as the water absorbent layer (14) becomes wetter.

2. A label (10) according to claim 1, wherein the water absorbent layer (14) comprises a fibrous pad; or wherein the water absorbent layer (14) comprises a hydrophilic material; or wherein the water absorbent layer (14) comprises a sponge material.

3. A label (10) according to claim 1 or 2, wherein the base layer (12) is larger than the water absorbent layer (14) such that a display region (15) extends beyond the periphery of the water absorbent layer (14).

4. A label (10) according to any preceding claim, wherein the water absorbent layer (14) is located centrally with respect to the base layer (12).

5. A label (10) according to any preceding claim, wherein the base layer (12) comprises polypropylene; and/or wherein the water absorbent layer (14) is adhered to the base layer (12) with a pressure sensitive adhesive.

6. A label (10) according to any preceding claim, wherein the adhesive layer (30) comprises a pressure sensitive adhesive.

7. A plurality of labels (10), each in accordance with any preceding claim, releasably adhered to a backing sheet.

8. A wound reel of labels (10) comprising a wound backing sheet with a plurality of labels (10), each in accordance with any preceding claim, releasably adhered to the backing sheet.

9. Product packaging defining a volume for containing a product, wherein a self-adhesive moisture control label (10) in accordance with any one of claims 1 to 6 is adhered to the packaging such that the label is disposed within the volume so that it can control the humidity of the volume.

10. Product packaging according to claim 9, wherein the product packaging is packaging for a tobacco product; and/or wherein the product packaging comprises a tub (100) having a container base defining a volume and a detachably attachable lid (104), wherein the label (10) is adhered to the inside of the lid (104).

## Patentansprüche

1. Selbstklebendes Feuchtigkeitsregulierungsetikett (10) für Produktverpackungen, das Folgendes umfasst:
eine wasserabsorbierende Schicht (14), die Wasser absorbieren kann; und
eine Klebstoffschicht (30), die so vorgesehen ist, dass das Etikett (10) an die Verpackung geklebt werden kann;
wobei das Feuchtigkeitsregulierungsetikett (10) die Feuchtigkeit der Atmosphäre innerhalb der Verpackung regulieren kann;
wobei von der wasserabsorbierenden Schicht (14) absorbiertes Wasser von der wasserabsorbierenden Schicht (14) verdampfen kann, um die Feuchtigkeit der Atmosphäre innerhalb der Verpackung zu regulieren; wobei das selbstklebende Feuchtigkeitsregulierungsetikett eine Basisschicht (12) umfasst, auf deren Vorderseite die wasserabsorbierende Schicht (14) geklebt ist, und wobei die Klebstoffschicht (30) auf der Rückseite der Basisschicht (102) vorgesehen ist; **dadurch gekennzeichnet, dass** ein graphisches Zeichen (26) so auf der Basisschicht angeordnet ist, dass die wasserabsorbierende Schicht (14) in einem trockenen Zustand eine erste Opazität aufweist und dass die wasserabsorbierende Schicht (14) in einem wassergesättigten Zustand eine zweite Opazität aufweist, die geringer ist als die erste Opazität, so dass das graphische Zeichen (14) beim Gebrauch weniger sichtbar wird, wenn die wasserabsorbierende Schicht (14) trockener wird, und sichtbarer wird, wenn die wasserabsorbierende Schicht (14) feuchter wird.

2. Etikett (10) nach Anspruch 1, wobei die wasserabsorbierende Schicht (14) ein Faserkissen umfasst; oder wobei die wasserabsorbierende Schicht (14) ein hydrophiles Material umfasst; oder wobei die wasserabsorbierende Schicht (14) ein Schwammmaterial umfasst.

3. Etikett (10) nach Anspruch 1 oder 2, wobei die Basisschicht (12) größer ist als die wasserabsorbierende Schicht (14), so dass sich eine Anzeigeregion (15) über die Peripherie der wasserabsorbierenden Schicht (14) hinaus erstreckt.

4. Etikett (10) nach einem vorherigen Anspruch, wobei die wasserabsorbierende Schicht (14) in Bezug auf die Basisschicht (12) mittig angeordnet ist.

5. Etikett (10) nach einem vorherigen Anspruch, wobei die Basisschicht (12) Polypropylen umfasst; und/oder wobei die wasserabsorbierende Schicht (14) mit einem druckempfindlichen Klebstoff auf die Basisschicht (12) geklebt ist.

6. Etikett (10) nach einem vorherigen Anspruch, wobei die Klebstoffschicht (30) einen druckempfindlichen Klebstoff umfasst.

7. Mehrzahl von Etiketten (10), jeweils nach einem vorherigen Anspruch, die ablösbar auf einen Trägerbogen geklebt sind.

8. Gewickelte Etikettenrolle (10), die einen gewickelten Trägerbogen mit einer Mehrzahl von Etiketten (10), jeweils nach einem vorherigen Anspruch, umfasst, die lösbar auf den Trägerbogen geklebt sind.

9. Produktverpackung, die ein Volumen zur Aufnahme eines Produkts definiert, wobei ein selbstklebendes Feuchtigkeitsregulierungsetikett (10) nach einem der Ansprüche 1 bis 6 so auf die Verpackung geklebt ist, dass das Etikett innerhalb des Volumens angeordnet ist, so dass es die Feuchtigkeit des Volumens regulieren kann.

10. Produktverpackung nach Anspruch 9, wobei die Produktverpackung eine Verpackung für ein Tabakprodukt ist; und/oder wobei die Produktverpackung einen Becher (100) mit einer ein Volumen definierenden Behälterbasis und einem abnehmbar aufzusetzenden Deckel (104) umfasst, wobei das Etikett (10) auf die Innenseite des Deckels (104) geklebt ist.

## Revendications

1. Etiquette autocollante de régulation d'humidité (10) pour un emballage de produit, comprenant :
une couche d'absorption d'eau (14) capable d'absorber l'eau ; et
une couche adhésive (30) permettant de coller l'étiquette (10) contre l'emballage,
dans laquelle l'étiquette de régulation d'humidité (10) est capable de réguler l'humidité de l'atmosphère à l'intérieur de l'emballage ;
dans laquelle l'eau absorbée par la couche d'absorption d'eau (14) peut s'évaporer de la couche d'absorption d'eau (14) de manière à réguler l'humidité de l'atmosphère à l'intérieur de l'emballage; dans laquelle l'étiquette autocollante de régulation de l'humidité comprend une couche de base (12) sur la face avant de laquelle est collée la couche d'absorption d'eau (14) et dans laquelle la couche adhésive (30) est disposée sur la face arrière de la couche de base (12) ;
**caractérisé en ce que**
un indice graphique (26) est apposé sur la couche de base ;
**en ce que**, dans un état sec, la couche d'absorption d'eau (14) présente une première opacité et **en ce que**, dans un état saturé d'eau, la couche d'absorption d'eau (14) présente une deuxième opacité inférieure à la première opacité, de telle sorte qu'en cours d'utilisation, l'indice graphique (14) devienne moins visible à mesure que la couche d'absorption d'eau (14) devient plus sèche, et devienne plus visible à mesure que la couche d'absorption d'eau (14) devient plus humide.

2. Etiquette (10) selon la revendication 1, dans laquelle la couche d'absorption d'eau (14) comprend un tampon fibreux, ou dans laquelle la couche d'absorption d'eau (14) comprend une matière hydrophile, ou dans laquelle la couche d'absorption d'eau (14) comprend une matière spongieuse.

3. Etiquette (10) selon la revendication 1 ou 2, dans laquelle la couche de base (12) est plus grande que la couche d'absorption d'eau (14) de telle sorte qu'une zone d'affichage (15) s'étende au-delà de la périphérie de la couche d'absorption d'eau (14).

4. Etiquette (10) selon l'une quelconque des revendications précédentes, dans laquelle la couche d'absorption d'eau (14) est située centralement par rapport à la couche de base (12).

5. Etiquette (10) selon l'une quelconque des revendications précédentes, dans laquelle la couche de base (12) comprend du polypropolène ; et/ou dans laquelle la couche d'absorption d'eau (14) est collée contre la couche de base (12) avec un adhésif sensible à la pression.

6. Etiquette (10) selon l'une quelconque des revendications précédentes, dans laquelle la couche adhésive (30) comprend un adhésif sensible à la pression.

7. Pluralité d'étiquettes (10), chacune conforme à n'importe quelle revendication précédente, collée de manière détachable contre une feuille de support.

8. Bobine d'étiquettes enroulées (10) comprenant une feuille de support enroulée comportant une pluralité d'étiquettes (10), chacune conforme à l'une quelconque des revendications précédentes, adhérant de manière détachable à la feuille de support.

9. Emballage de produit définissant un volume destiné à contenir un produit, dans lequel une étiquette autocollante de régulation d'humidité (10) conforme à l'une quelconque des revendications 1 à 6 est collée contre l'emballage de telle sorte que l'étiquette soit disposée dans le volume afin qu'elle puisse réguler l'humidité du volume.

10. Emballage de produit selon la revendication 9, dans lequel l'emballage de produit est un emballage pour un produit de tabac ; et/ou dans lequel l'emballage de produit comprend un pot (100) présentant une base de récipient définissant un volume et un couvercle détachable (104), dans lequel l'étiquette (10) est collée contre l'intérieur du couvercle (104).
